# EUROPÄISCHE PATENTSCHRIFT

(11) **EP 1 227 802 B1**
(45) Veröffentlichungstag und Bekanntmachung des Hinweises auf die Patenterteilung: **15.03.2006**
(21) Anmeldenummer: 00975993.7
(22) Anmeldetag: 03.11.2000
(51) Int. Cl.: A61K 9/70, A61K 9/20

(54) **FILMFÖRMIGE ZUBEREITUNG ZUR BIPHASIGEN FREISETZUNG PHARMAKOLOGISCH WIRKSAMER ODER ANDERER SUBSTANZEN**
FILM PREPARATION FOR BIPHASIC RELEASE OF PHARMACOLOGICALLY ACTIVE OR OTHER SUBSTANCES
PREPARATION SOUS FORME DE FILM POUR LIBERATION SELON DEUX PHASES DE SUBSTANCES PHARMACOLOGIQUEMENT ACTIVES OU D'AUTRES SUBSTANCES

(30) Priorität: 12.11.1999 DE 19954421
(43) Veröffentlichungstag der Anmeldung: 07.08.2002
(60) Teilanmeldung: 05026782.2
(73) Patentinhaber: LTS LOHMANN Therapie-Systeme AG, 56626 Andernach (DE)
(72) Erfinder: LUDWIG, Karin, 56589 Datzeroth (DE); KRUMME, Markus, 56567 Neuwied (DE)
(74) Vertreter: Flaccus, Rolf-Dieter
(86) Internationale Anmeldenummer: PCT/EP2000/010861
(87) Internationale Veröffentlichungsnummer: WO 2001/035934

(56) Entgegenhaltungen:
- WO-A-96/41617
- US-A- 4 765 983
- US-A- 5 085 865
- US-A- 5 393 528
- US-A- 5 681 583

## Beschreibung

Die Erfindung betrifft eine filmförmige Zubereitung, welche eine biphasige Freisetzung von darin enthaltenen Stoffen an eine flüssige Umgebung ermöglicht. Ferner schließt die Erfindung ein Verfahren zur Herstellung solcher Zubereitungen mit ein, sowie deren Verwendung zur Freisetzung von Stoffen, insbesondere von Arzneistoffen, an Körperflüssigkeiten des menschlichen oder tierischen Organismus.

Bei Anwendungen, welche auf einer gezielten Abgabe oder Freisetzung von Wirk- oder Inhaltsstoffen an das umgebende Milieu beruhen, kommt es in gewissen Fällen - beispielsweise bei der Verabreichung von Medikamenten aus Depot-Arzneiformen - darauf an, daß anfangs eine hohe Initialdosis freigesetzt wird, gefolgt von einer niedrigeren, aber über einen gewissen Zeitraum hinweg konstanten Folge- oder Erhaltungsdosis. Dadurch soll bewirkt werden, daß sehr schnell ein hoher Wirkstoffspiegel in den Körperflüssigkeiten aufgebaut wird, der anschließend durch die konstante Verabreichung der Erhaltungsdosis auf einem gewünschten Wert gehalten werden kann.

Um eine schnelle Freisetzung zu erreichen, muß eine Zubereitung oder Formulierung beispielsweise eines Arzneistoffs derart ausgestaltet sein, daß sie eine im Verhältnis zum Volumen große Oberfläche aufweist. Auf diese Weise bleiben die Diffusionswege möglichst kurz, und eine Freisetzung des Wirkstoffs kann in sehr kurzer Zeit erfolgen. Außerdem sollte ein solches Freisetzungssystem möglichst kompakt sein, um ausreichende Festigkeit und gute Handhabbarkeit zu gewährleisten.

Eine filmförmige Darreichungsform, welche Wirkstoffe in gelöster, emulgierter oder suspendierter Form enthält, ermöglicht aufgrund der geringen Schichtdicke und der entsprechend kurzen Diffusionswege extrem niedrige Freisetzungszeiten und damit eine schnelle Abgabe von Initialdosen. Die Freisetzung des Wirkstoffs kann entweder in der Weise geschehen, daß dieser aus der filmförmigen Matrix herausdiffundiert, oder daß die Filmschicht aufgelöst oder zersetzt wird und der Wirkstoff dadurch in die Umgebung gelangt.

Es sind bereits wirkstoffhaltige Filme bekannt, deren Matrix auf hydrophilen Polymeren beruht. Zur Einstellung der physiko-chemischen Parameter des Films, sowie des Geschmacks oder der chemischen Stabilität können diese filmförmigen Darreichungsformen noch weitere Inhaltsstoffe oder Hilfsstoffe enthalten. Die Herstellung solcher Filme erfolgt im allgemeinen in der Weise, daß eine wirk- und hilfsstoffhaltige Lösung eines hydrophilen Polymers auf eine inerte Prozeßfolie beschichtet wird. Durch anschlie-ßende Trocknung wird das Lösungsmittel entzogen, und die wirkstoffhaltige Matrix bleibt als Film zurück.

Wie beschrieben, läßt sich auch mit nur einphasigen Darreichungsformen eine schnelle Wirkstofffreisetzung erreichen, allerdings kommt es dabei nach der Abgabe der Initialdosis meist zu einem unerwünschten raschen Absinken der Wirkung. Zudem besteht die Gefahr von Überdosierungen, oder - bei Wirkstoffen, welche durch eine gesteigerte, konzentrationsabhängige Elimination gekennzeichnet sind -, zu einem "wasted dose"-Szenario.

US-A-5 393 528 offenbart wirkstoffhaltige Filme, die Schichten mit unterschiedlichen Löslichkeits- und Wirkstofffreisetzungseigenschaften enthalten können. Um diese unterschiedlichen Eigenschaften zu erreichen, werden Polyvinylalkohole mit unterschiedlichen Löslichkeits-Eigenschaften verwendet, oder die Filme werden bei der Herstellung durch Einblasen von Gasen aufgeschäumt.

US-A-4 765 983 offenbart zweischichtige, wirkstoffhaltige mucoadhäsive Filme mit einer schnell zerfallenden Schicht und einer langsam löslichen Schicht, wodurch eine anfänglich schnelle Wirkstofffreisetzung mit einer anschließenden langsamen und andauernden Wirkstofffreisetzung kombiniert werden kann. Die beiden Schichten bestehen im wesentlichen aus einem wasserlöslichen Polymer oder einer Mischung solcher Polymere, wobei als wasserlösliche Polymere Polyvinylpyrrolidon, Polyvinylalkohol, Natrium-Carboxymethylcellulose, Methylcellulose, Hydroxypropylcellulose und andere in Betracht kommen. Die schnell löslichen Schichten und die langsam löslichen Schichten unterscheiden sich bezüglich ihrer Polymerzusammensetzung, wobei die schnell löslichen Schichten bevorzugt niedrigsubstituierte Hydroxypropylcellulose und die langsam löslichen Schichten bevorzugt hochsubstituierte Hydroxypropylcellulose enthalten.

Die vorliegende Erfindung beruht auf der Aufgabe, die im Zusammenhang mit der Verwendung von einphasigen Dosisformen auftretenden Nachteile zu vermeiden. Insbesondere bestand die Aufgabe darin, ein filmförmiges System bereitzustellen, welches eine zweiphasige Freisetzung von Inhaltsstoffen an ein flüssiges Milieu ermöglicht, und zwar dergestalt, daß zunächst eine hohe Initialdosis einer Substanz an die Umgebung abgegeben wird, und danach mit langsamerer Freisetzungsgeschwindigkeit eine weitere Substanz.

Speziell sollte ein derartiges System die Freisetzung von Arzneistoffen im oder am menschlichen oder tierischen Organismus ermöglichen. Zusätzlich zu der erwünschten Freisetzungscharakteristik muß ein solches System auch ausreichende mechanische Stabilität besitzen, um eine gute Handhabbarkeit zu ermöglichen. Ferner bestand die Aufgabe darin, ein möglichst einfaches, kostengünstiges und auf verschiedene Ausführungsvarianten adaptierbares Herstellungsverfahren aufzuzeigen.

überraschenderweise gelingt die Lösung dieser Aufgabe mit einer filmförmigen Zubereitung auf Polymer-Basis gemäß Anspruch 1, sowie mit Zubereitungen gemäß den Ansprüchen 2 bis 11, welche weitere nützliche Ausführungsvarianten der Erfindung verkörpern.

Die erfindungsgemäßen filmförmigen Zubereitungen sind dadurch gekennzeichnet, daß sie mindestens zwei Polymermatrixschichten umfassen, die sich hinsichtlich ihres Aufbaus aus Polymeren unterscheiden, wobei die Freisetzung aus einer der Schichten schnell erfolgt und die Freisetzung aus mindestens einer weiteren Schicht langsam erfolgt, und wobei die Polymermatrix der schnell freisetzenden Schicht auf Basis von Polyvinylalkohol(en) und die Polymermatrix der langsam freisetzenden Schicht auf Basis von Cellulose-Ether(n) hergestellt ist.
Vorzugsweise werden die unterschiedlichen Freisetzungsgeschwindigkeiten in den verschiedenen Filmschichten dadurch erzielt, daß die Matrix der schnell freisetzenden Schicht aus hydrophilen Polymeren oder Polymeren mit hoher Wasserlöslichkeit, oder aus Polymergemischen mit diesen Eigenschaften, aufgebaut ist, und daß die Matrix der langsam freisetzenden Schicht(en) aus schwerer oder nicht wasserlöslichen, weniger hydrophilen Polymeren oder Polymergemischen aufgebaut ist. Dadurch wird erreicht, daß sich die erstgenannte Schicht im Sinne einer "flash-release"-Formulierung in wäßriger Umgebung schnell auflöst.

Hingegen ist die langsam freisetzende Schicht aufgrund ihrer speziellen Polymerzusammensetzung relativ inert gegenüber wäßrigen Systemen und wird infolgedessen nur langsam aufgelöst. Deshalb erreicht sie trotz ihrer ebenfalls geringen Schichtdicke nur niedrige Freisetzungsraten. Auf diese weise wird ein System mit einer biphasigen Freisetzungscharakteristik ermöglicht, welches mit geringen Schichtdicken realisierbar ist und deshalb Vorteile beispielsweise bei der Herstellung und Handhabung aufweist.

Hinsichtlich der erzielbaren Freisetzungsgeschwindigkeiten sind solche Ausführungsvarianten der Erfindung besonders gut geeignet, bei denen die Lösungsgeschwindigkeit der schnell freisetzenden Schicht so eingestellt ist, daß eine Fläche von mindestens 10 cm² dieser Schicht in physiologischen Flüssigkeiten (oder in künstlichen Nachbildungen davon) in weniger als 15 min, bevorzugt in weniger als 5 min, besonders bevorzugt in weniger als 1 min aufgelöst wird.

Ferner sind solche Ausführungsvarianten der Erfindung besonders gut geeignet, bei denen die Lösungsgeschwindigkeit der langsam freisetzenden Schicht derartig eingestellt ist, daß für die Auflösung einer Fläche von maximal 10 cm² dieser Schicht in physiologischen Flüssigkeiten (oder künstlichen Nachbildungen davon) mindestens 15 min, bevorzugt mindestens 60 min, besonders bevorzugt mindestens 120 min benötigt werden.

Als Polymerbestandteile der schnell freisetzenden Schicht eignen sich vor allem Polymere oder Polymergemische, welche ausgewählt sind aus der Gruppe, die Cellulose-Ether, Polyvinylalkohol, Polyvinylacetat, Polyvinylpyrrolidon, Copolymerisate der vorgenannten Polymere, sowie Gelatine, Alginate und andere natürliche oder teilsynthetische Polymere umfaßt. Auch diverse andere, dem Fachmann bekannte Polymere synthetischen, teilsynthetischen und auch natürlichen Ursprungs können verwendet werden.

Besonders bevorzugt sind filmförmige Zubereitungen, bei denen die Matrix der schnell freisetzenden Schicht aus Polyvinylalkohol oder aus Polyvinylalkohol enthaltenden Polymergemischen aufgebaut ist.

Bei einer weiteren bevorzugten Ausführungsform ist die Matrix der schnell freisetzenden Schicht aus Cellulose-Ethern, bevorzugt Hydroxypropylmethyl-Cellulose, oder aus Gemischen von Cellulose-Ethern aufgebaut.

Für den Aufbau der Matrix der langsam freisetzenden Schicht(en) kommen vorzugsweise Polymere zum Einsatz, welche ausgewählt sind aus der Gruppe, die Cellulose-Ether, bevorzugt Ethylcellulose, sowie Polyvinylalkohol, Polyurethan, Polymethacrylate, Polymethylmethacrylate und Derivate und Copolymerisate der vorgenannten Polymere umfaßt.

Die niedrige Löslichkeit oder Unlöslichkeit des Polymerfilms in wäßrigem Milieu, oder auch dessen wasserresistente Ausgestaltung, hat zur Folge, daß die Wirkstoffabgabe nur langsam auf dem Diffusionswege erfolgt, mit - bei geeigneter Formulierung - niedrigem Diffusionskoeffizienten. Dies bewirkt eine langsame Wirkstoffabgabe.

Um die Löslichkeit bzw. die Freisetzungsgeschwindigkeit der langsam freisetzenden Schicht(en) zu verringern, kann die Polymerschicht einer Temperung unterworfen werden. So kann beispielsweise ein hochhydrolysierter Polyvinylalkohol als Basispolymer für die nicht lösliche, langsam freisetzende Schicht eingesetzt werden, wenn dieser durch Temperung unlöslich gemacht wird.

Zusätzlich kann die Wirkstofffreisetzung auch durch weitere galenische Maßnahmen verzögert werden, beispielsweise durch Kornvergrößerung der Wirkstoffpartikel, durch Coating der Wirkstoffpartikel oder durch Mikroverkapselung. Auch für die nicht pharmakologisch aktiven Inhaltsstoffe läßt sich mit derartigen Methoden eine Freisetzungsverzögerung herbeiführen.

Die erfindungsgemäßen filmförmigen Zubereitungen haben vorzugsweise eine Dicke im Bereich zwischen 5 und 500 µm, besonders bevorzugt im Bereich zwischen 10 und 100 µm.
Der oder die in der Film-Polymermatrix enthaltenen Wirkstoffe sind in Form einer echten (molekulardispersen) oder kolloidalen Lösung, einer Emulsion oder einer Suspension in den mindestens zwei Schichten verteilt.

Zusätzlich können noch Hilfs- oder Zusatzstoffe anwesend sein, welche beispielsweise die Stabilität der Zubereitung erhöhen, die Freisetzungsrate modulieren oder die Resorption oder Permeation des Wirkstoffs im menschlichen oder tierischen Organismus verbessern können. Zu den Hilfs- oder Zusatzstoffen zählen unter anderem auch Zerfallsförderer, Weichmacher, Netzmittel, Strukturgeber und Texturmodifizierer.

Bevorzugte Ausführungsformen der erfindungsgemäßen filmförmigen Zubereitungen enthalten in mindestens einer der Schichten einen pharmakologisch aktiven Wirkstoff oder mehrere solcher Wirkstoffe. Die zweite Schicht oder - bei einem mehrschichtigen Aufbau - die weiteren Schichten können entweder einen Aroma-, Geschmacks-, Süß- oder sonstigen Inhaltsstoff, oder ebenfalls einen Arzneistoff enthalten. Letzterer kann auch mit dem Arzneistoff der ersten Schicht identisch sein. Auch Kombinationen verschiedener Arzneistoffe oder andere Inhaltsstoffe können sich als sinnvoll erweisen.

Neben der Möglichkeit, einen Wirkstoff (oder mehrere Wirkstoffe) in zwei aufeinanderfolgenden Phasen zunächst schnell und danach langsam abzugeben, ist die räumliche Trennung in verschiedene wirkstoffhaltige Kompartimente in Gestalt mehrerer Schichten auch aus anderen Gründen vorteilhaft. So ist es möglich, zwei oder mehrere Wirkstoffe, welche nicht miteinander kompatibel sind, in getrennten Schichten zu kombinieren. Ferner können der Wirkstoff oder die Wirkstoffe auch von zusätzlich vorhandenen Aromastoffen, Süßstoffen etc. räumlich abgetrennt werden. Auf diese Weise können einige Unverträglichkeiten umgangen werden, und es sind neuartige Kombinationen von Wirkstoffen untereinander sowie mit weiteren Inhaltsstoffen möglich, welche in einer erfindungsgemäßen mehrschichtigen filmförmigen Zubereitung gemeinsam verabreicht werden können.

Grundsätzlich eignen sich die erfindungsgemäßen filmförmigen Zubereitungen immer dann, wenn ein oder mehrere Stoffe in einem biphasigem Schema, gekennzeichnet durch eine anfänglich schnelle und nachfolgend langsame Freisetzung, an eine flüssige Umgebung abgegeben werden sollen. Bevorzugt werden die erfindungsgemäßen Systeme eingesetzt, um Inhaltsstoffe, insbesondere pharmakologisch aktive Stoffe, an Körperflüssigkeiten abzugeben. Hierzu werden die filmförmigen Zubereitungen in Körperöffnungen, Körperhöhlen oder das Körperinnere von Menschen oder tierischen Organismen appliziert. Unter dem Einfluß der umgebenden Körperflüssigkeiten (z. B. Speichel, Magensaft) kommt es sodann zu der vorbestimmten, unterschiedlich schnellen Auflösung der einzelnen Schichten, wodurch die schnelle bzw. langsame Freisetzung der pharmazeutischen Wirkstoffe oder sonstigen Inhaltsstoffe herbeigeführt wird.

Selbstverständlich eignen sich die filmförmigen Zubereitungen auch zur Abgabe von Substanzen an synthetische Nachbildungen von Körperflüssigkeiten. Aufgrund ihrer Fähigkeit, Arzneistoffe mit einer biphasigen Freisetzungscharakteristik an Körperflüssigkeiten abzugeben, können die erfindungsgemäßen Filmzubereitungen vorteilhaft für eine medikamentöse Therapie oder Prophylaxe bei den verschiedensten Krankheitserscheinungen eingesetzt werden.

Für die Herstellung der biphasig freisetzenden filmförmigen Zusammensetzungen können verschiedene erfindungsgemäß vorgeschlagene Verfahrensvarianten zum Einsatz kommen. Vorzugsweise wird man bei der Herstellung so vorgehen, daß man zunächst eine filmbildende Lösung herstellt, welche die zur Herstellung schnell löslicher bzw. freisetzender Schichten geeigneten Polymere oder Polymergemische enthält, und der man die freizusetzenden Wirk- oder sonstigen Inhaltsstoffe zusetzt. Ebenfalls können - sofern erforderlich - weitere Hilfs- oder Zusatzstoffe hinzugefügt werden.

Die so erhaltene Lösung, welche eine für die weitere Verarbeitung geeignete Viskosität aufweisen muß, wird anschließend durch Rakel-, Walzenauftrags- oder Sprühverfahren auf eine inerte Unterlage beschichtet und getrocknet, wodurch das Lösungsmittel entzogen und ein Film gebildet wird (die jeweils zuerst hergestellte Schicht wird im folgenden auch als "Primärfilm" bezeichnet). Die geeigneten inerten Unterlagen sind dem Fachmann bekannt und können beispielsweise in Form von Prozeßfolien oder Metallbändern ausgebildet sein. Es sind sowohl kontinuierliche Beschichtungsverfahren möglich, wie auch diskontinuierliche Verfahren, wobei eine Beschichtung grundsätzlich auf beliebige inerte Oberflächen erfolgen kann.

Für die langsam freisetzende Schicht wird auf analoge Weise eine Lösung hergestellt, die ein geeignetes Polymer oder Polymergemisch sowie Wirk- und / oder Inhaltsstoffe und gegebenenfalls Hilfs- und Zusatzstoffe enthält. Bei der Auswahl der Lösungsmittel ist zu beachten, daß diese die Primärschicht nicht angreifen dürfen. Die Lösung wird durch eines der vorstehend genannten Beschichtungsverfahren oder auch durch Bedruckung auf den vorbereiteten Primärfilm aufgetragen und nachfolgend getrocknet, wodurch ein Filmverbund oder Laminat entsteht. Auf entsprechende Weise können noch weitere langsam freisetzende Schichten hinzugefügt werden. Ebenso ist es möglich, die zweite oder folgende Schicht auf einem inerten Träger bereitzustellen und anschließend auf die Primärschicht zu laminieren.

Bei einer besonders vorteilhaften Variante des Herstellungsverfahrens ist die Reihenfolge der Verfahrensschritte verändert, dergestalt, daß zunächst die schlecht lösliche oder langsam freisetzende Polymerlösung als "Primärschicht" auf einen inerten Träger aufgetragen wird. Auf diese Schicht wird nach erfolgter Trocknung die gut lösliche bzw. schnell freisetzende Filmschicht aufgetragen. Diese Vorgehensweise ermöglicht es beispielsweise, einen hochhydrolysierten Polyvinylalkohol als Basispolymer für die schwer bzw. nicht lösliche Schicht einzusetzen, der durch Temperung unlöslich gemacht wird. Der zweite (wasserlösliche, schnell freisetzende) Film kann z. B. durch teilhydrolysierten Polyvinylalkohol formuliert werden, der auf den getemperten (Primär-)Film aufgeschichtet wird.

Eine weitere Variante des erfindungsgemäßen Herstellungsverfahrens sieht vor, daß der Primärfilm aus einer Polymerschmelze erzeugt wird, die durch Rakel-, Walzenauftrags-, Sprüh- oder Extrusionsverfahren auf eine inerte Unterlage beschichtet und nachfolgend gekühlt und / oder getrocknet wird. Die zweite oder die weiteren Schichten werden anschließend aus Lösungen, wie oben beschrieben, auf diese Primärschicht aufgetragen, wobei Lösungsmittel verwendet werden, die die Primärschicht nicht angreifen oder auflösen.

Die Erfindung wird durch die nachfolgenden Beispiele näher erläutert.

### Beispiele

### Beispiel 1

Teilhydrolysierter Polyvinylalkohol niedriger Viskosität (Mowiol® 8-88, Fa. Clariant) wird in heißem Wasser gelöst. Durch Zusatz geeigneter Hilfsstoffe wie Weichmachern, Zerfallsförderern, Netzmitteln und ähnlichen dem Fachmann bekannten Additiven sowie den Wirkstoffen stellt man daraus eine viskose Masse her, die auf eine inerte Unterlage beschichtet wird.
Nach Trocknung erhält man einen gut handhabbaren Film ("Primärfilm"), der in Wasser leicht löslich ist und
28,6 Gew.-% Mowiol® 8-88,
7.9 Gew.-% Titandioxid,
37,2 Gew.-% Siliciumdioxid,
11,5 Gew.-% Polyethylenglykol 400,
4,6 Gew.-% Polyethylenglykol 4000 und
10,2 Gew.-% Sorbitol enthält.

Auf entsprechende Weise stellt man eine zweite Masse basierend auf Ethylcellulose her, die in Ethanol gelöst wird und die ebenfalls Wirkstoffe sowie geeignete Hilfsstoffe ähnlich der ersten Schicht enthält, nämlich
ca. 57 Gew.-% Ethylcellulose,
ca. 5 Gew.-% Lanette O (wachsartige Salbengrundlage, enthaltend Cetylstearylalkohol, in einer Mischung aus 1-Hexadecanol 1-Octadecanol)
ca. 30 Gew.-% Siliciumdioxid sowie
ca. 8 Gew.-% Titandioxid.

Die Primärschicht (Mowiol® 8-88) ist nicht in Ethanol löslich und verhält sich daher inert gegenüber der zweiten Beschichtungsmasse, die auf den Mowiolfilm aufgetragen werden kann. Der Verbund wird getrocknet und man erhält eine biphasig freisetzende Filmformulierung (= filmförmige Zubereitung).

Bei filmförmigen Zubereitungen (auch "wafer" genannt) der Größe 15 x 15 mm können hierbei durch geeignete Formulierungen in vitro-Freisetzungszeiten erreicht werden, welche - wie beabsichtigt - für die schnell lösliche Schicht im Bereich von kürzer als 60 s und für die langsam lösliche Schicht im Bereich von wenigen Stunden liegen.

### Beispiel 2: Rezepturen für biphasige Systeme.

Biphasig freisetzender Polymerfilm mit dem Wirkstoff Coffeincitrat.

Gemäß dem in Beispiel 1 angegebenen Herstellungsverfahren wurde ein biphasig freisetzendes System (filmförmige Zubereitung) mit dem Wirkstoff Coffein hergestellt. Die erste Beschichtungsmasse bzw. die zweite Beschichtungsmasse hatten in diesem Fall die folgende Zusammensetzung:
Beschichtungsmasse für die erste Schicht (Primärfilm):
   "BMX 0001": 50,41 Gew.-% Feststoffgehalt
      7,14 Gew.-% Titandioxid
      25,88 Gew.-% Polyvinylalkohol
      9,52 Gew.-% Coffeincitrat
      4,13 Gew.-% Polyethylenglykol 4000
      10,35 Gew.-% Polyethylenglykol 400
      9,32 Gew.-% Sorbitol
      33,65 Gew.-% Siliciumdioxid
      Wasser als Lösemittel.
Beschichtungsmasse für die zweite Schicht:
   "BMX 0002": 22,49 Gew.-% Feststoffgehalt
      86,16 Gew.-% Ethylcellulose
      4,31 Gew.-% Cetylstearylalkohol
      9,53 Gew.-% Coffeincitrat
      Ethanol als Lösungsmittel

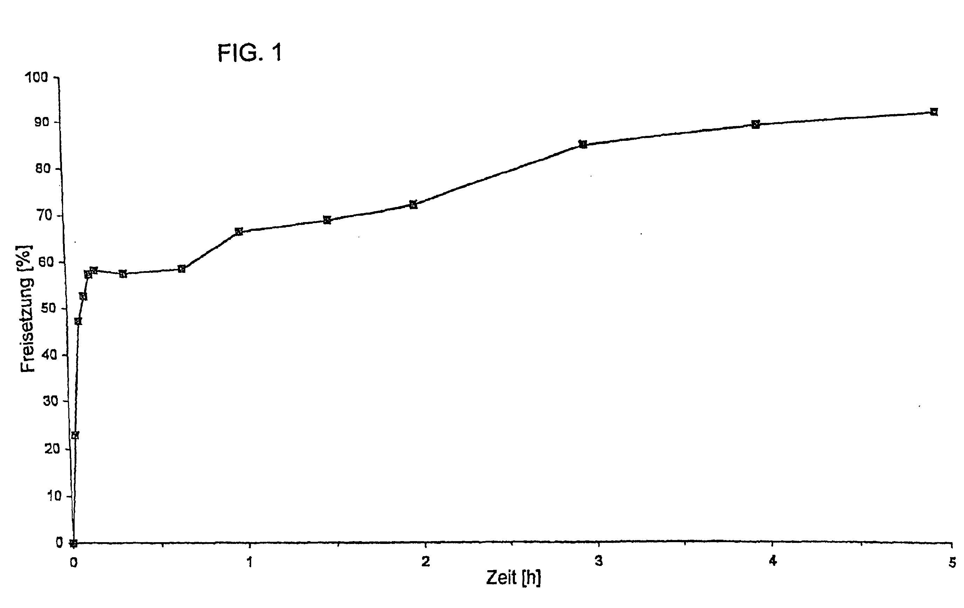

Mit diesen Beschichtungsmassen wurden, wie für Beispiel 1 beschrieben, filmförmige Zubereitungen ("wafer") hergestellt und es wurde die Freisetzung von Coffein aus diesen filmförmigen Zubereitungen in Wasser untersucht.
Die Versuchsergebnisse sind in Fig. 1 graphisch dargestellt.
Fig. 1 zeigt die Freisetzung von Coffein aus erfindungsgemäßen filmförmigen Zubereitungen ("wafer") in Wasser. Aus der in Fig. 1 gezeigten Freisetzungskurve kann man sehr gut das zweiphasige Abgabeverhalten erkennen: Eine schnelle Freisetzung ("initial burst") innerhalb von etwa 10 Minuten sowie eine langsame, aber stetige und kontrollierte Freisetzung über den verbleibenden Zeitraum (im konkreten Fall: 5 Stunden).
Die angegebenen Freisetzungen wurden mit "Paddle-over-disc" und "Rotary-basket"-Methoden ermittelt. Die einzelnen Punkte der Freisetzungskurve sind Mittelwerte aus Messungen mit verschiedenen Meßmethoden.

## Patentansprüche

1. Filmförmige Zubereitung auf Polymer-Basis zur biphasigen Freisetzung von darin enthaltenen Substanzen an eine flüssige Umgebung, **dadurch gekennzeichnet, daß** die Zubereitung mindestens zwei Polymermatrixschichten umfaßt, die sich hinsichtlich ihres Aufbaus aus Polymeren unterscheiden, wobei die Freisetzung aus einer der Schichten schnell erfolgt und die Freisetzung aus mindestens einer weiteren Schicht langsam erfolgt, und wobei die Polymermatrix der schnell freisetzenden Schicht auf Basis von Polyvinylalkohol (en) und die Polymermatrix der langsam freisetzenden Schicht auf Basis von Cellulose-Ether(n) hergestellt ist.

2. Filmförmige Zubereitung nach Anspruch 1 **dadurch gekennzeichnet, daß** mindestens eine der Polymermatrixschichten einen oder mehrere pharmazeutische Wirkstoffe enthält.

3. Filmförmige Zubereitung nach Anspruch 1 oder 2, **dadurch gekennzeichnet, daß** sie in mindestens einer der Polymermatrixschichten eine oder mehrere freisetzbare Substanzen enthält, die ausgewählt sind aus der Gruppe, die Geschmacksstoffe, Aromastoffe, Süßstoffe und Substanzen mit erfrischender Wirkung umfaßt.

4. Filmförmige Zubereitung nach einem oder mehreren der vorangehenden Ansprüche, **dadurch gekennzeichnet, daß** es sich bei der umgebenden Flüssigkeit, in welche die Substanzen freigesetzt werden, um physiologische Flüssigkeiten, oder künstlichen Nachbildungen davon, von Mensch oder Tier handelt.

5. Filmförmige Zubereitung nach einem oder mehreren der vorangehenden Ansprüche, **dadurch gekennzeichnet, daß** die Lösungsgeschwindigkeit der schnell freisetzenden Schicht derartig eingestellt ist, daß eine Fläche von mindestens 10 cm² dieser Schicht in physiologischen Flüssigkeiten oder künstlichen Nachbildungen davon in weniger als 15 min, bevorzugt in weniger als 5 min, besonders bevorzugt in weniger als 1 min aufgelöst wird.

6. Filmförmige Zubereitung nach einem oder mehreren der vorangehenden Ansprüche, **dadurch gekennzeichnet, daß** die Lösungsgeschwindigkeit der langsam freisetzenden Schicht derartig eingestellt ist, daß für die Auflösung einer Fläche von maximal 10 cm² dieser Schicht in physiologischen Flüssigkeiten oder künstlichen Nachbildungen mindestens 15 min, bevorzugt mindestens 60 min, besonders bevorzugt mindestens 120 min benötigt werden.

7. Filmförmige Zubereitung nach einem oder mehreren der vorangehenden Ansprüche, **dadurch gekennzeichnet, daß** die Matrix der schnell freisetzenden Schicht Polymere enthält, welche ausgewählt sind aus der Gruppe, die Cellulose-Ether, insbesondere Hydroxypropylmethyl-Cellulose, Gemische von Cellulose-Ethern, Polyvinylacetat, Polyvinylpyrrolidon, Copolymerisate der vorgenannten Polymere, sowie Gelatine, Alginate und andere natürliche oder teilsynthetische Polymere umfaßt.

8. Filmförmige Zubereitung nach Anspruch 7, **dadurch gekennzeichnet, daß** die Matrix der schnell freisetzenden Schicht aus Polyvinylalkohol enthaltenden Polymergemischen aufgebaut ist.

9. Filmförmige Zubereitung nach einem oder mehreren der vorangehenden Ansprüche, **dadurch gekennzeichnet, daß** die Matrix der langsam freisetzenden Schicht(en) Polymere enthält, welche ausgewählt sind aus der Gruppe, die Ethylcellulose, sowie Polyvinylalkohol, Polyurethan, Polymethacrylate, Polymethylmethacrylate und Derivate oder Copolymerisate der vorgenannten Polymere umfaßt.

10. Filmförmige Zubereitung nach einem oder mehreren der vorangehenden Ansprüche, **dadurch gekennzeichnet, daß** die Matrix der langsam freisetzenden Schicht (en) einen Gehalt an getempertem Polyvinylalkohol aufweist.

11. Filmförmige Zubereitung nach einem oder mehreren der vorangehenden Ansprüche, **dadurch gekennzeichnet, daß** sie eine Dicke im Bereich zwischen 5 und 500 µm, besonders bevorzugt im Bereich zwischen 10 und 100 µm, aufweist.

12. Verfahren zur Herstellung einer filmförmigen Zubereitung nach einem oder mehreren der Ansprüche 1 bis 11, **gekennzeichnet durch** folgende Arbeitsachritte:
a) Herstellung der für die schnell freisetzende Schicht geeigneten Polymerlösung unter Hinzufügen der freizusetzenden Substanzen;
b) Beschichtung dieser Lösung auf eine inerte Unterlage durch Rakel-, Walzenauftrags- oder Sprühverfahren und nachfolgende Trocknung, wodurch die schnell freisetzende Filmschicht gebildet wird;
c) Herstellung der für die langsam freisetzende Schicht geeigneten Polymerlösung unter Hinzufügen der freizusetzenden Substanzen;
d) Beschichtung dieser Lösung auf die schnell freisetzende Filmschicht **durch** Rakel-, Walzenauftrags-, Sprühverfahren oder Bedruckung und anschließende Trocknung, wodurch die langsam freisetzende Filmschicht gebildet wird.

13. Verfahren nach Anspruch 12, **dadurch gekennzeichnet, daß** in den Schritten a) und b) zunächst die langsam freisetzende Filmschicht hergestellt wird und diese sodann gemäß den Schritten c) und d) mit der schnell freisetzenden Filmschicht beschichtet wird.

14. Verfahren nach Anspruch 12 oder 13, **dadurch gekennzeichnet, daß** bei der Herstellung der ersten Filmschicht von einer Polymerschmelze ausgegangen wird, welche durch Rakel-, Walzenauftrags-, Sprüh- oder Extrusionsverfahren auf eine inerte Unterlage aufgetragen und anschließend getrocknet und / oder gekühlt wird, und daß danach die Beschichtung der weiteren Schichten aus Polymerlösungen erfolgt.

15. Verfahren nach einem der Ansprüche 12 bis 14, **dadurch gekennzeichnet, daß** die Freisetzung der Substanz(en) aus der/den langsam freisetzenden Schicht(en) durch Kornvergrößerung der Stoffpartikel, durch Coating oder durch Mikroverkapselung der Substanz oder des Wirkstoffs, oder durch ähnliche wirkende galenische Maßnahmen weiter verzögert wird.

16. Verwendung einer filmförmigen Zubereitung nach einem oder mehreren der Ansprüche 1 bis 11 zur Freisetzung von Substanzen, vorzugsweise von pharmazeutischen Wirkstoffen, in menschliche oder tierische Körperflüssigkeiten oder künstliche Nachbildungen solcher Körperflüssigkeiten.

17. Verwendung einer filmförmigen Zubereitung nach einem oder mehreren der Ansprüche 1 bis 11 zur Herstellung von Arzneizubereitungen zur medikamentösen therapeutischen Behandlung von Krankheiten beim Menschen oder bei Tieren.

## Claims

1. A polymer-based preparation in film form for biphasic release of substances contained therein to liquid surroundings, **characterized in that** the preparation comprises at least two polymer matrix layers which differ in terms of their construction from polymers, with release taking place rapidly from one of the layers and release taking place slowly from at least one other layer, and the polymer matrix of the rapidly releasing layer being based on polyvinyl alcohol(s) and the polymer matrix of the slowly releasing layer being based on cellulose ether(s).

2. A preparation in film form according to Claim 1, **characterized in that** at least one of the polymer matrix layers contains one or more active pharmaceutical ingredients.

3. A preparation in film form according to claim 1 or 2, **characterized in that** it contains in at least one of the polymer matrix layers one or more releasable substances which are selected from the group comprising flavorings, odorizers, sweeteners and substances with a refreshing effect.

4. A preparation in film form according to one or more of the preceding claims, **characterized in that** the surrounding liquid into which the substances are released comprises physiological fluids, or artificial simulations thereof, of humans or animals.

5. A preparation in film form according to one or more of the preceding claims, **characterized in that** the rate of dissolution of the rapidly releasing layer is adjusted so that an area of at least 10 cm² of this layer is dissolved in physiological fluids or artificial simulations thereof in less than 15 min, preferably in less than 5 min, particularly preferably in less than 1 min.

6. A preparation in film form according to one or more of the preceding claims, **characterized in that** the rate of dissolution of the slowly releasing layer is adjusted so that at least 15 min, preferably at least 60 min, particularly preferably at least 120 min, are required for the dissolution of an area not exceeding 10 cm² of this layer in physiological fluids or artificial simulations.

7. A preparation in film form according to one or more of the preceding claims, **characterized in that** the matrix of the rapidly releasing layer contains polymers which are selected from the group which comprises cellulose ethers, particularly hydroxypropyl methyl cellulose, mixtures of cellulose ethers, polyvinyl acetate, polyvinylpyrrolidone, copolymers of the aforementioned polymers, and gelatin, alginates and other natural or partially synthetic polymers.

8. A preparation in film form according to claim 7, **characterized in that** the matrix of the rapidly releasing layer is composed of polyvinyl alcohol-containing polymer mixtures.

9. A preparation in film form according to one or more of the preceding claims, **characterized in that** the matrix of the slowly releasing layer(s) contains polymers which are selected from the group which comprises ethylcellulose, and polyvinyl alcohol, polyurethane, polymethacrylates, poly(methyl methacrylates) and derivatives or copolymers of the aforementioned polymers.

10. A preparation in film form according to one or more of the preceding claims, **characterized in that** the matrix of the slowly releasing layer(s) has a content of heat-treated polyvinyl alcohol.

11. A preparation in film form according to one or more of the preceding claims, **characterized in that** it has a thickness in the range between 5 and 500 µm, particularly preferably in the range between 10 and 100 µm.

12. A process for the production of a preparation in film form according to one or more of claims 1 to 11, **characterized by** the following steps:
a) production of the polymer solution suitable for the rapidly releasing layer, with addition of the substances to be released;
b) coating of this solution onto an inert substrate by knife or roller application or spraying processes and subsequent drying to form the rapidly releasing film layer;
c) production of the polymer solution suitable for the slowly releasing layer, with addition of the substances to be released;
d) coating of this solution onto the rapidly releasing film layer by knife or roller application or spraying processes or printing and subsequent drying to form the slowly releasing film layer.

13. A process according to claim 12, **characterized in that** in steps a) and b) there is initial production of the slowly releasing film layer, and this is then coated with the rapidly releasing film layer as in steps c) and d).

14. A process according to claim 12 or 13, **characterized in that** production of the first film layer starts from a polymer melt which is applied by knife or roller application, spraying or extrusion processes to an inert substrate and is then dried and/or cooled, and **in that** the further layers are coated from polymer solutions.

15. A process according to any of claims 12 to 14, **characterized in that** the release of the substance(s) from the slowly releasing layer(s) is further delayed by increasing the size of the substance particles, by coating or by microencapsulation of the substance or of the active ingredient, or by pharmaceutical technology measures having similar effects.

16. The use of a preparation in film form according to one or more of claims 1 to 11 for the release of substances, preferably active pharmaceutical ingredients, into human or animal body fluids or artificial simulations of such body fluids.

17. The use of a preparation in film form according to one or more of claims 1 to 11 for the production of pharmaceutical preparations for medical therapeutic treatment of diseases in humans or animals.

## Revendications

1. Préparation sous forme de film à base de polymères pour la libération biphasée de substances qui y sont contenues dans un environnement liquide, **caractérisée en ce que** la préparation comprend au moins deux couches à matrice polymère, qui se distinguent en ce qui concerne leur constitution polymère, la libération d'une des couches étant rapide et la libération de ladite au moins une autre couche étant lente et la matrice polymère de la couche à libération rapide étant à base de poly(alcool(s) vinylique(s)) et la matrice polymère de la couche à libération lente étant à base d'éther(s) de cellulose.

2. Préparation sous forme de film selon la revendication 1, **caractérisée en ce qu'**au moins une des couches à matrice polymère contient une ou plusieurs substances actives pharmaceutiques.

3. Préparation sous forme de film selon la revendication 1 ou 2, **caractérisée en ce qu'**elle contient, dans au moins une des couches à matrice polymère, une ou plusieurs substances libérables, qui sont choisies dans le groupe comprenant les agents gustatifs, les arômes, les édulcorants et les substances à effet rafraîchissant.

4. Préparation sous forme de film selon l'une ou plusieurs des revendications précédentes, **caractérisée en ce qu'**il s'agit, pour le liquide environnant, dans lequel les substances sont libérées, de liquides physiologiques ou d'imitations synthétiques de ceux-ci de l'homme ou de l'animal.

5. Préparation sous forme de film selon l'une ou plusieurs des revendications précédentes, **caractérisée en ce que** la vitesse de solubilisation de la couche à libération rapide est réglée de telle manière qu'une surface d'au moins 10 cm² de cette couche est dissoute dans les liquides physiologiques ou les imitations synthétiques de ceux-ci en moins de 15 minutes, de préférence en moins de 5 minutes, de manière particulièrement préférée en moins de 1 minute.

6. Préparation sous forme de film selon l'une ou plusieurs des revendications précédentes, **caractérisée en ce que** la vitesse de solubilisation de la couche à libération lente est réglée de telle manière qu'il faut, pour la dissolution d'une surface d'au maximum 10 cm² de cette couche dans des liquides physiologiques ou des imitations synthétiques de ceux-ci, au moins 15 minutes, de préférence au moins 60 minutes, de manière particulièrement préférée au moins 120 minutes.

7. Préparation sous forme de film selon l'une ou plusieurs des revendications précédentes, **caractérisée en ce que** la matrice de la couche à libération rapide contient des polymères qui sont choisis dans le groupe comprenant les éthers de cellulose, en particulier l'hydroxypropylméthylcellulose, les mélanges d'éthers de cellulose, le poly(acétate de vinyle), la polyvinylpyrrolidone, les copolymères des polymères susmentionnés ainsi que les gélatines, les alginates et d'autres polymères naturels ou semi-synthétiques.

8. Préparation sous forme de film selon la revendication 7, **caractérisée en ce que** la matrice de la couche à libération rapide comprend des mélanges de polymères contenant du poly(alcool vinylique).

9. Préparation sous forme de film selon l'une ou plusieurs des revendications précédentes, **caractérisée en ce que** la matrice de la ou des couches à libération lente contient des polymères qui sont choisis dans le groupe qui comprend l'éthylcellulose, ainsi que le poly(alcool vinylique), le polyuréthane, les polyméthacrylates, les poly(méthacrylates de méthyle) et les dérivés ou les copolymères des polymères susmentionnés.

10. Préparation sous forme de film selon l'une ou plusieurs des revendications précédentes, **caractérisée en ce que** la matrice de la ou des couches à libération lente présente une teneur en poly(alcool vinylique) traité thermiquement.

11. Préparation sous forme de film selon l'une ou plusieurs des revendications précédentes, **caractérisée en ce qu'**elle présente une épaisseur dans la plage entre 5 et 500 µm, de manière particulièrement préférée dans la plage entre 10 et 100 µm.

12. Procédé pour la réalisation d'une préparation sous forme de film selon l'une ou plusieurs des revendications 1 à 11, **caractérisé par** les étapes de travail suivantes :
a) préparation de la solution de polymères appropriée pour la couche à libération rapide avec addition des substances à libérer;
b) revêtement de cette solution sur un substrat inerte par des procédés à la racle, d'application au cylindre ou par pulvérisation et séchage consécutif, ce qui forme la couche de film à libération rapide;
c) préparation de la solution de polymères appropriée pour la couche à libération lente avec addition des substances à libérer ;
d) revêtement de cette solution sur la couche de film à libération rapide par des procédés à la racle, d'application au cylindre, par pulvérisation ou par impression et séchage consécutif, ce qui forme la couche de film à libération lente.

13. Procédé selon la revendication 12, **caractérisé en ce que** dans les étapes a) et b), on prépare d'abord la couche de film à libération lente, puis on la revêt selon les étapes c) et d) de la couche de film à libération rapide.

14. Procédé selon la revendication 12 ou 13, **caractérisé en ce que** lors de la préparation de la première couche de film, on part d'une masse fondue de polymère, qui est appliquée par un procédé à la racle, d'application au cylindre, par pulvérisation ou par extrusion sur un substrat inerte qui est ensuite séchée et/ou refroidie, puis on procède au revêtement des autres couches à partir de solutions de polymères.

15. Procédé selon l'une quelconque des revendications 12 à 14, **caractérisé en ce que** la libération de la ou des substances de la ou des couches à libération lente est ralentie davantage par une augmentation de la granulométrie des particules de la substance, par revêtement ou par micro-encapsulage de la substance ou de la substance active ou par des mesures galéniques agissant de manière analogue.

16. Utilisation d'une préparation sous forme de film selon l'une ou plusieurs des revendications 1 à 11 pour la libération de substances, de préférence de substances actives pharmaceutiques, dans les liquides corporels humains ou animaux ou dans des imitations synthétiques de ces liquides corporels.

17. Utilisation d'une préparation sous forme de film selon l'une ou plusieurs des revendications 1 à 11 pour la réalisation de préparations médicamenteuses pour le traitement thérapeutique médicamenteux de maladies chez les hommes ou les animaux.
